# EUROPEAN PATENT SPECIFICATION

(11) **EP 1 797 831 B1**
(45) Date of publication and mention of the grant of the patent: **30.06.2010**
(21) Application number: 06025705.2
(22) Date of filing: 12.12.2006
(51) Int. Cl.: A61B 17/11

(54) **Compression anastomosis device**
Vorrichtung für Kompressionsanastomose
Dispositif pour réaliser une anastomose par compression

(30) Priority: 15.12.2005 US 304400
(43) Date of publication of application: 20.06.2007
(73) Proprietor: Tyco Healthcare Group LP, Norwalk, CT 06856 (US)
(72) Inventor: Fowler, David N., Cheshire, CT 06410 (US)
(74) Representative: Alcock, David

(56) References cited:
- WO-A-81/00668
- US-A- 4 055 186
- US-A- 4 766 898
- US-A- 5 234 448
- US-A- 5 527 324
- US-A- 5 562 690
- US-A- 5 697 943

## Description

### BACKGROUND

### Technical Field

The present disclosure relates generally to devices for anastomosing tissue. More particularly, the present disclosure relates to a compression anastomosis device for use in minimally invasive surgical procedures.

### Description of the Related Art

An anastomotic device formed of separate ring members having a plurality of fenestrated projections connected by a separate coupling tube is discussed in U.S. Pat. No. 3,974,835. In the anastomotic device disclosed in this patent, the free ends of the tube to be joined are tied to the separate ring members at the fenestrated projections, and the singular coupling tube connects the two ring members to engage the tubular ends in a relationship that will enable them to grow together permanently and thereby approximate the diameter of the outer surface of the tubular member.

A similar device formed of a singular pliable, unitary cylindrical sleeve made of knit fabric is disclosed in U.S. Pat. No. 4,182,339. In this patent, the unitary, knit cylindrical sleeve has its ends rolled outwardly upon themselves to form relatively firm ring members in spaced relationship which are then connected to the ends of the tubular members in a configuration that will enable these members to grow together.

The above-referenced patents disclose structure to draw the ends of the tubular members together by either turning or rolling these members inwardly to facilitate healing because they enable the ends to rest in a contiguous relationship. Other techniques involve devices like those disclosed in U.S. Pat. Nos. 3,496,939 and 3,254,650.

WO 81/00668 discloses a medical device for connecting two portions of the intestine comprising the features defined in the preamble of appended claim 1, whereby the device comprises two coupling components which are connected via a magnetic fixing means located on each coupling component.

US 5,527,324 discloses a surgical stent for use in supporting the walls of a tubular organ during anastomosis. The stent includes a single tubular body having a diameter adapted to be about equal to the diameter of the organ to be anastomosed and a central ridge of greater diameter than the tubular organ. The ridge everts the ends of the tubular organ to facilitate suturing.

US 4,055,186 discloses an anastomosis button formed of a pair of axially engaged complementary clamping members, each clamping member including a hub and a crown.

It is desirable that a nonpermanent connector or junction device be used to join the vessel ends in anastomotic surgery since a permanent connector will tend to prevent the changes in diameter which are necessary for the proper functioning of the intestine. Any foreign substance used in anastomotic surgery ideally should partially or completely disintegrate, bio-absorb and/or bio-resorb once the vessel ends have partially or fully healed.

Compression anastomotic devices have been developed in the past for receiving the free ends of anatomic tubular structures to be anastomosed. An example of such an anastomotic device has been developed by Tyco Healthcare LP, and is currently sold under the trademark VALTRAC^{®}. This assembly includes a pair of ring members, each ring member for securement to the free end of each tubular structure to be anastomosed. Each ring member has a connecting structure which mates with the other ring member to connect the ring members. U.S. Patent No. 4,766,898 issued to Hardy et al., currently owned by Tyco Healthcare LP, describes the operating features of such an anastomotic device.

### SUMMARY

The present disclosure is directed to an anastomotic device as defined in claim 1 for use in the surgical joining of the free ends of a first and a second tubular structure to be anastomosed. The device includes a first and second ring member, each of which is secured to a free end of a tubular structure. Each ring member includes a cylindrical outer portion (first outer portion and second outer portion) and an inner portion (first inner portion and second inner portion) which are operatively attached to each other (i.e., first outer portion is operatively attached to first inner portion; second outer portion is operatively attached to second inner portion). The outer portions are each open ended cylinders and have holes disposed around their perimeters for facilitating tissue growth therethrough. The inner portions, also open ended, have connecting means and are designed to matingly engage one another.

During an anastomosis procedure, the tubular structures are joined together and secured at their ends. The cylindrical outer portions of the anastomotic device of the present disclosure provide support for the portions of the tubular structures near and adjacent the ends of the tubular structure, thus reducing the likelihood of stricture during healing of the anastomotic tissue. Further, the cylindrical outer portions maintain a generally constant diameter of the tubular structure. Such a lumen of constant diameter is beneficial for fluid and/or gas to flow through. The holes on the outer portion provide a scaffold, which enable tissue to grow therethrough. This in-growth of tissue further enhances the strength of the portions of the tissue near the anastomosis.

In operation, each ring member is inserted into a tubular structure to be anastomosed. The tissue is tied, via a purse-string or similar arrangement, around the outer portion of each ring member. The ring members are then brought together until the inner portions meet and mate with each other, thus locking the ring members together and forming a flow path therethrough. When the inner portions are mated, the tissue around the outer portions of the anastomotic device are contiguously positioned in a manner that will enable them to grow together permanently. The cylindrical outer portions maintain the desired diameter of the lumen during healing. The holes on the outer portion provide a scaffold for tissue to grow into, thus strengthening the anastomosis site and contiguous areas.

Over time, the tissue surrounding the anastomosis site will heal and the anastomotic device will be absorbed by and discharged through the body. Once healing is complete, the two tubular structures will have joined together and produced a continuous and strong lumen. Generally, the anastomotic device may remain in the body from about 14 to about 21 days before it is discharged.

As mentioned above, the anastomosis device will be constructed of a bioabsorbable material. The anastomosis device may comprise bioabsorbable polymeric resin such as, for example, a copolymer of polylactic acid (PLA) and polyglycolic acid (PGA). The relative proportion of the components may be chosen to suit the surgical application. For example, under identical processing conditions, polyglycolic acid is typically the stronger of the two components and more crystalline. However, polyglycolic acid is more rapidly absorbed by body tissue. Hence for surgical applications where it is desired to maintain the implant strength over a longer period of time, the fiber will typically contain more polylactic acid. The fibers can be fibers of the type used in manufacturing suture material. Additionally, several other materials for forming this device are disclosed in U.S. Patent No. 3,297,033 and are referred to as poly-hydroxyacetic ester and lactide co-polymers. The materials disclosed in the above-referenced patent constitute a partial list of possible materials as molded surgical articles made from a wide range of glycolide /lactide copolymers have been known and utilized for many years.

The length of the cylindrical outer portion may be in the range of about three centimeters to about four centimeters, but may be longer or shorter depending on the desired support to be provided to the tissue and the desired tissue ingrowth. This length provides a desirable amount of support to help resist the tubular structure from stricture and it also helps to maintain the diameter of the lumen during healing.

The outer portions may have a relatively thin wall thickness. Such a minimal wall thickness, while still providing support for tissue, may make the outer cylindrical portions flexible. Since the thickness of the walls corresponds to the depth of the holes around their perimeters, a minimal wall thickness would also facilitate the growth of tissue therethrough.

The inner diameter of the first inner portion is substantially the same as the inner diameter of the second inner portion. The outer diameter of the first outer portion is substantially the same as the outer diameter of the second outer portion which is approximately equal to the inner diameter of the tubular vessel to be anastomosed. Accordingly, the anastomotic device can be designed to fit into any suitable tubular vessel.

In one embodiment, the outer portions are at least partially comprised of a mesh-like material.

### BRIEF DESCRIPTION OF THE DRAWINGS

Embodiments of the present disclosure are described hereinbelow with reference to the drawings wherein:
FIGS. 1 and 2 are prior art figures of an anastomotic device;
FIGS. 3-8 are perspective views of the anastomotic device of the present disclosure illustrated at different stages of the process by which the first and second ring members are inserted into and join tubular structures;
FIG. 9 is an enlarged view of the first and second ring members illustrated in an unmated arrangement;
FIG. 10 is an enlarged view of the first and second ring members illustrated in a mated arrangement;
FIG. 11 is a cross-section view of the first and second ring members inserted into tubular structures illustrated in an unmated arrangement; and
FIG. 12 is a cross-section view of the first and second ring members inserted into tubular structures illustrated in a mated arrangement.

### DETAILED DESCRIPTION

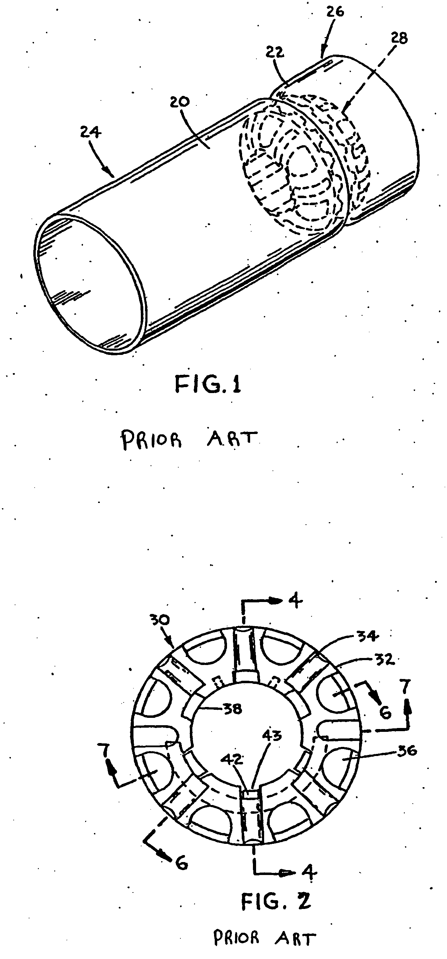

Figs. 1 and 2, designated "Prior Art," illustrate the anastomotic device of U.S. Patent No. 4,766,898. Fig. 1 illustrates an anastomotic device 28 connecting free ends 20, 22 of two tubular tissue members 24, 26. Fig. 2 shows one of a pair of ring members 32 having a plurality of slots 34 and apertures 36 about its periphery and a mating prong 38 for matingly corresponding with other ring member. Each mating prong 38 carries a plurality of locking slots 43 designed and positioned to mate cooperatively with engaging pawls 42. When ring members 32 are joined, a single device 30 is formed and has a substantially toroidal shape.

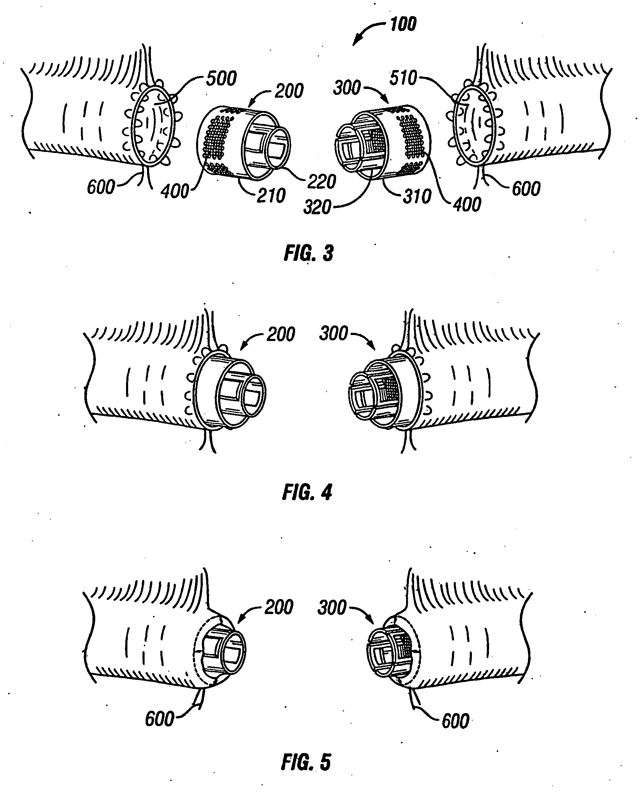

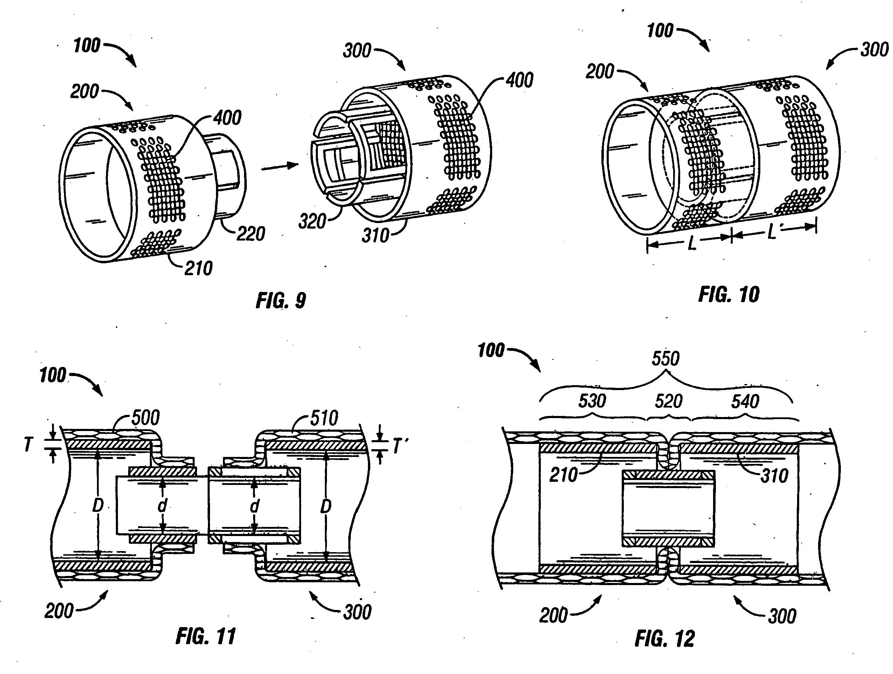

The anastomosis device of the present disclosure is used to join vessel ends in anastomotic surgery and is generally referenced by number 100, and is generally shown in Figs. 3-12. As best shown in Figs. 3 and 9, the anastomosis device 100 is generally comprised of a first ring member 200 and a second ring member 300. First ring member 200 is generally comprised of outer portion 210 and first inner portion 220. Second ring member 300 is generally comprised of second outer portion 310 and second inner portion 320. Outer portions 210, 310 are operatively attached to inner portions 220, 320, respectively.

Outer portions 210, 310 are cylindrical ring-like structures with a length and a diameter and inner portions 220, 320 are ring-like structures with a length and a diameter. The diameter of first outer portion 210 is substantially the same as the diameter of second outer portion 310, hereinafter referred to as outer portion diameter D (Fig. 11). The diameter of first inner portion 220 is substantially the same as the diameter of second inner portion 320, hereinafter referred to as inner portion diameter d (Fig. 11). Inner portions 220, 320 are appropriately sized to fit at least partially within outer portions 210, 310, respectively, thus outer portion diameter D is greater than inner portion diameter d. The diameters D of outer portions 210, 320 are sized to fit within the tissue lumen 550 and may also be sized to substantially equal an outer diameter of a surgical device (not shown) used to position such an anastomotic device 100. Desirably, the inner diameters d of inner portions 220, 320 are dimensioned and configured to be slightly smaller than the diameters D of outer portions 210, 310. Such a configuration provides a passageway through the tissue lumen 550.

First outer portion 210 and second outer portion 310 have lengths L, L' (length of cylinder), respectively. Lengths L, L' may have a range of about three centimeters to about four centimeters, and may be longer or shorter depending on the desired support to be provided to the tissue 530, 540 and the desired tissue ingrowth. Thicknesses T, T' of first outer portion 210 and second outer portion 310 (Figs. 10 and 11), respectively, may be dimensioned and configured to be as thin as possible while still providing support for tissue 530, 540. In a particularly useful embodiment, these thicknesses T, T' allow for outer portions 210, 310 to be flexible. Outer portions 210, 310 provide support for the anastomosis along their lengths L, L', respectively. Specifically, site of anastomosis 520 is surrounded by tissue 530, 540 near and adjacent thereto. This tissue 530, 540 is supported by cylindrical outer portions 210, 310, respectively (see Fig. 12). This support provided by the length L, L' of cylindrical outer portions 210, 310 maintains the desired circumference of lumen 550 during healing.

A plurality of holes 400 is annularly disposed around the perimeter of each outer portion 210, 310. Plurality of holes 400 provides a scaffold, which enables tissue 530, 540 near and adjacent anastomosis site 520 to grow therethrough. Tissue 530, 540 grows through plurality of holes 400 and naturally attaches to itself on inside of outer portions 210, 310, thus at least partially encapsulating outer portions 210, 310. This in-growth of tissue and partial encapsulation of outer portions 210, 310 enhances the strength of the portions of the tissue 530, 540 near anastomosis site 520 and further helps to maintain the circumference of lumen 550 during healing.

Inner portions 220, 320 are configured and dimensioned to mate with each other via a snap-fit or similar type of locking feature, including a bayonet or turn-lock. In a particularly useful embodiment, it is not necessary to turn the ring members 200, 300 to connect them to each other. Accordingly, when inner portions 220, 320 are mated, first ring member 200 and second ring member 300 are locked together. An example of similar matingly corresponding locking features are disclosed in U.S. Patent No. 4,755,898 issued to Hardy et al.

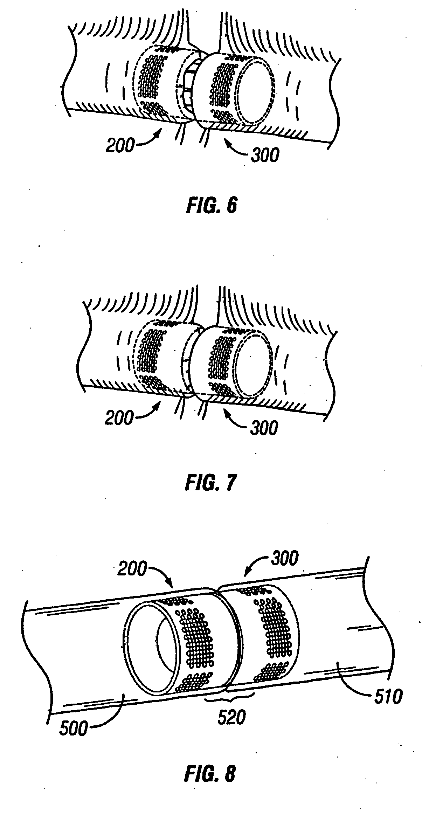

In operation, first ring member 200 is inserted into a first tubular structure 500 and second ring member 300 is inserted into a second tubular structure 510. Several stages of the process by which first and second ring members 200, 300 are inserted into and join tubular structures 500, 510 are depicted in the illustrations of Figs. 3-8. Figs. 9 and 11 show anastomosis device 100 in an unapproximated position, and Figs. 10 and 12 show anastomosis device 100 approximated. The tissue of tubular structures 500, 510 is tied, via a purse-string 600 or similar arrangement, around outer portion 210, 310 of each ring member 200, 300, respectively. Ring members 200, 300 are then brought together until inner portions 220, 320 meet and mate with each other. When inner portions 220, 320 are mated, tubular structures 500, 510 around outer portions 210, 310 of anastomotic device 100 are contiguously positioned and compressed between each ring member 200, 300 (Fig. 8) in a manner that will enable them to grow together permanently into a single lumen 550 (Fig. 12). Further, a path through inner portions 220, 320 for bodily fluid to flow through is established when two ring members 200, 300 of anastomotic device 100 are locked together (see Fig. 12).

Anastomotic device 100 may be fabricated from any bioresorbable polymer or copolymer known to those skilled in the art, so long as the polymer utilized has sufficient strength and possesses the necessary mechanical properties to permit formation. Suitable polymers which may be utilized to form anastomotic device 100 include, but are not limited to, trimethylene carbonate, caprolactone, dioxanone, glycolic acid, lactic acid, glycolide, lactide, homopolymers thereof, copolymers thereof, and combinations thereof. Over time, the tissue 530, 540 surrounding the anastomosis site 520 will heal and the anastomotic device 100 will be at least partially absorbed by and then discharged through the body. Once healing is complete, the two tubular structures 500, 510 will have joined together and produced a continuous and strong lumen 550 for fluid and/or gas to flow through. In a particularly useful embodiment, the anastomotic device 100 is partially disintegrable, absorbable or resorbable such that the anastomotic device 100 softens and separates from the body. The anastomotic device 100 is then passed through the body with waste. The anastomotic device 100 may be constructed to break into a plurality of pieces that are passed through the body separately. The anastomotic device 100 may remain in the body from about 14 to about 21 days before it is passed though the body.

While the above description contains many specifics, these specifics should not be construed as limitations on the scope of the present disclosure, but merely as exemplifications of preferred embodiments thereof. Those skilled in the art will envision many other possible variations that are within the scope of the present disclosure.

## Claims

1. An anastomotic device (100) for use in the surgical joining of a first free end of a first tubular structure and a second free end of a second tubular structure to be anastomosed, the anastomotic device comprising:
a first ring member (200) adapted for securement to the first free end of first tubular structure, the first ring member including
a first inner portion (220) having connecting means disposed thereon, and
a first outer portion (210) having a length and a thickness, the first outer portion being substantially cylindrically shaped, having open ends and having a plurality of holes (400) disposed therethrough, the first outer portion being operatively attached to the first inner portion; and
a second ring member (300) adapted for securement to the second free end of second tubular structure, the second ring member including
a second inner portion (320) having connecting means disposed thereon for cooperatively mating with the connecting means of the first inner portion, and
a second outer portion (310) having a length and a thickness, the second outer portion being substantially cylindrically shaped, having open ends and having plurality of holes (400) disposed therethrough, the second outer portion being operatively attached to the second inner portion,
wherein the first inner portion (220) and the second inner portion (320), when mated with each other, form a flow path through the anastomotic device,
**characterized in that** the plurality of holes disposed through the first outer portion and the second outer portion form a mesh-like structure provide a scaffold which facilitates tissue to grow through the first outer portion and through the second outer portion.

2. The anastomotic device (100) according to claim 1, wherein the length of the first outer portion (210) is between about three centimeters and about four centimeters.

3. The anastomotic device (100) according to claim 1 or 2, wherein the length of the second outer portion (310) is between about three centimeters and about four centimeters.

4. The anastomotic device (100) according to claim 1, 2 or 3, wherein the first ring member (200) and the second ring member (300) are made of a bioabsorbable material.

5. The anastomotic device (100) according to any one of the preceding claims, wherein the length of the first outer portion (210) and the length of the second outer portion (310) are configured and dimensioned to resist stricture of the first tubular structure and the second tubular structure when the first inner portion (220) and the second inner portion (320) are mated.

6. The anastomotic device (100) according to any one of the preceding claims and so constructed that the first ring member (200) locks with the second ring member (300) when the first inner portion (220) is caused matingly to co-operate with the second inner portion (320).

7. The anastomotic device (100) according to any one of the preceding claims, wherein an inside diameter of the first inner portion (220) is substantially the same as an inside diameter of the second inner portion (320).

8. The anastomotic device (100) of any one of the preceding claims, wherein the first ring member (200) and the second ring member (300) are comprised of a mesh-like material.

## Patentansprüche

1. Anastomotische Vorrichtung (100) zur Verwendung bei der operativen Verbindung eines ersten freien Endes einer ersten röhrenartigen Struktur und eines zweiten freien Endes einer zweiten röhrenartigen Struktur, die anastomisiert werden sollen, wobei die anastomotische Vorrichtung aufweist:
ein erstes Ringelement (200), das zum Sichern des ersten freien Endes der ersten röhrenartigen Struktur eingerichtet ist, wobei das erste Ringelement umfasst:
einen ersten inneren Abschnitt (220) mit einem daran angeordnetem Verbindungsmittel und
einen ersten äußeren Abschnitt (210) mit einer Länge und einer Dicke, wobei der erste äußere Abschnitt im Wesentlichen zylindrisch geformt ist mit offenen Enden und mit einer Mehrzahl von Löchern (400) darin angeordnet, wobei der erste äußere Abschnitt wirksam mit dem ersten inneren Abschnitt verbunden ist, und
ein zweites Ringelement (300), das zur Sicherung des zweiten freien Endes der zweiten röhrenartigen Struktur eingerichtet ist, wobei das zweite Ringelement umfasst:
einen zweiten inneren Abschnitt (320) mit einem Verbindungsmittel, das daran zum zusammenwirkenden Ineinandergreifen mit dem Verbindungsmittel des ersten inneren Abschnitts angeordnet ist, und
einem zweiten äußeren Abschnitt (310) mit einer Länge und einer Dicke, wobei der zweite äußere Abschnitt im Wesentlichen zylindrisch geformt ist mit offenen Enden und mit einer Mehrzahl von darin angeordneten Löchern (400), wobei der zweite äußere Abschnitt wirksam an dem zweiten inneren Abschnitt befestigt ist,
wobei der erste innere Abschnitt (220) und der zweite innere Abschnitt (320), wenn sie miteinander in Eingriff sind, einen Durchflusspfad durch die anastomotische Vorrichtung bilden,
**dadurch gekennzeichnet, dass** die Mehrzahl von in dem ersten äußeren Abschnitt und dem zweiten äußeren Abschnitt angeordneten Löchern eine gitterähnliche Struktur bilden, ein Gerüst bereitstellen, das es Gewebe ermöglicht, durch den ersten äußeren Abschnitt durch den zweiten äußeren Abschnitt hindurch zu wachsen.

2. Anastomotische Vorrichtung (100) nach Anspruch 1, **dadurch gekennzeichnet, dass** die Länge des ersten äußeren Abschnitts (210) zwischen ungefähr 3 cm und ungefähr 4 cm beträgt.

3. Anastomotische Vorrichtung (100) gemäß Anspruch 1 oder 2, **dadurch gekennzeichnet, dass** die Länge des zweiten äußeren Abschnitts (310) zwischen ungefähr 3 cm und ungefähr 4 cm beträgt.

4. Anastomotische Vorrichtung (100) nach Anspruch 1, 2 oder 3, **dadurch gekennzeichnet, dass** das erste Ringelement (200) und das zweite Ringelement (300) aus einem bioabsorbierbaren Material hergestellt sind.

5. Anastomotische Vorrichtung (100) nach einem der vorhergehenden Ansprüche, **dadurch gekennzeichnet, dass** die Länge des ersten äußeren Abschnitts (210) und die Länge des zweiten äußeren Abschnitts (310) so eingerichtet und dimensioniert sind, dass sie einer Verengung der ersten röhrenartigen Struktur und der zweiten röhrenartigen Struktur widerstehen, wenn der erste innere Abschnitt (220) und der zweite innere Abschnitt (320) miteinander in Eingriff sind.

6. Anastomotische Vorrichtung (100) nach einem der vorhergehenden Ansprüche und so konstruiert, dass das erste Ringelement (200) mit dem zweiten Ringelement (300) verrastet, wenn der erste innere Abschnitt (220) in Eingriff tretend veranlasst wird, mit dem zweiten inneren Abschnitt (320) zusammenzuwirken.

7. Anastomotische Vorrichtung (100) nach einem der vorhergehenden Ansprüche, **dadurch gekennzeichnet, dass** ein Innendurchmesser des ersten inneren Abschnitts (220) im Wesentlichen der gleiche ist wie ein Innendurchmesser des zweiten inneren Abschnitts (320).

8. Anastomotische Vorrichtung (100) nach einem der vorhergehenden Ansprüche, **dadurch gekennzeichnet, dass** das erste Ringelement (200) und das zweite Ringelement (300) ein gitterähnliches Material aufweisen.

## Revendications

1. Dispositif anastomotique (100) destiné à être utilisé dans la jonction chirurgicale d'une première extrémité libre d'une première structure tubulaire et d'une seconde extrémité libre d'une seconde structure tubulaire devant être anastomosées, le dispositif anastomotique comprenant :
un premier élément annulaire (200) conçu pour être fixé à la première extrémité libre de la première structure tubulaire, le premier élément annulaire comprenant une première partie interne (220) sur laquelle sont disposés des moyens de liaison, et
une première partie externe (210) ayant une longueur et une épaisseur, la première partie externe étant de forme sensiblement cylindrique, ayant des extrémités ouvertes et ayant une pluralité de trous (400) disposés à travers elle, la première partie externe étant fixée de façon opérationnelle à la première partie interne ; et
un second élément annulaire (300) conçu pour être fixé à la seconde extrémité libre de la seconde structure tubulaire, le second élément annulaire comprenant une seconde partie interne (320) sur laquelle sont disposés des moyens de liaison pour accouplement coopératif avec les moyens de liaison de la première partie interne, et
une seconde partie externe (310) ayant une longueur et une épaisseur, la seconde partie externe étant sensiblement de forme cylindrique, ayant des extrémités ouvertes et ayant une pluralité de trous (400) disposés à travers elle, la seconde partie externe étant fixée de façon opérationnelle à la seconde partie interne,
dans lequel la première partie interne (220) et la seconde partie interne (320) lorsqu'elles sont accouplées l'une à l'autre, forment une trajectoire d'écoulement à travers le dispositif anastomotique
**caractérisé en ce que** la pluralité de trous disposés à travers la première partie externe et la seconde partie externe forment une structure de type maille, offrent un échafaudage qui permet au tissu de se développer à travers la première partie externe et à travers la seconde partie externe.

2. Dispositif anastomotique (100) selon la revendication 1, dans lequel la longueur de la première partie externe (210) est comprise entre environ trois centimètres et environ quatre centimètres.

3. Dispositif anastomotique (100) selon la revendication 1 ou 2, dans lequel la longueur de la seconde partie externe (310) est comprise entre environ trois centimètres et environ quatre centimètres.

4. Dispositif anastomotique (100) selon la revendication 1, 2 ou 3, dans lequel le premier élément annulaire (200) et le second élément annulaire (300) sont constitués d'un matériau bioabsorbable.

5. Dispositif anastomotique (100) selon l'une quelconque des revendications précédentes, dans lequel la longueur de la première partie externe (210) et la longueur de la seconde partie externe (310) sont configurées et dimensionnées pour résister à la striction de la première structure tubulaire et de la seconde structure tubulaire lorsque la première partie interne (220) et la seconde partie interne (320) sont accouplées,

6. Dispositif anastomotique (100) selon l'une quelconque des revendications précédentes et conçu de telle sorte que le premier élément annulaire (200) se bloque avec le second élément annulaire (300) lorsque la première partie interne (220) est amenée à coopérer par accouplement avec la seconde partie interne (320).

7. Dispositif anastomotique (100) selon l'une quelconque des revendications précédentes, dans lequel un diamètre interne de la première partie interne (220) est sensiblement identique à un diamètre interne de la seconde partie interne (320).

8. Dispositif anastomotique (100) selon l'une quelconque des revendications précédentes, dans lequel le premier élément annulaire (200) et le second élément annulaire (300) sont constitués d'un matériau de type maille.
